# EUROPEAN PATENT SPECIFICATION

(11) **EP 1 898 808 B1**
(45) Date of publication and mention of the grant of the patent: **19.10.2016**
(21) Application number: 06745023.9
(22) Date of filing: 25.05.2006
(51) Int. Cl.: A61B 17/22

(54) **A ROTATIONAL ATHERECTOMY DEVICE AND METHOD OF USE**
DREHATHEREKTOMIEVORRICHTUNG UND VERWENDUNGSVERFAHREN
DISPOSITIF D'ATHERECTOMIE ROTATIF ET PROCEDE D'UTILISATION CORRESPONDANT

(30) Priority: 26.05.2005 GB 0510777
(43) Date of publication of application: 19.03.2008
(73) Proprietor: Cardiovascular Systems, Inc., St. Paul, MN 55112 (US)
(72) Inventor: Shturman, Leonid, deceased (CH)
(74) Representative: Viering, Jentschura & Partner mbB Patent- und Rechtsanwälte
(86) International application number: PCT/IB2006/051670
(87) International publication number: WO 2006/126175

(56) References cited:
- US-A- 5 314 438
- US-A- 5 681 336
- US-A1- 2004 158 270

## Description

The invention relates to devices for removing material from the interior of tubular structures. More specifically, the invention relates to a device for removing or reducing occlusions, and other unwanted deposits from the interior of blood vessels or other tubular structures by rotating an abrasive element (e.g., burr) within the structure to partially or completely eliminate the unwanted material.

Atherosclerosis, the clogging of arteries, is a leading cause of coronary heart disease. Blood flow through the peripheral arteries (e.g., carotid, femoral, renal, etc.), is similarly affected by the development of atherosclerotic blockages. One existing method of removing or reducing blockages in blood vessels is known as rotational or ablative atherectomy. A long guidewire is inserted into the desired blood vessel and across the stenotic lesion, and a hollow drive shaft is advanced over the guidewire. The distal end of the drive shaft terminates in a burr formed from solid material such as brass and which is provided with an abrasive surface such as diamond grit or diamond particles. The burr is positioned against the occlusion, and the drive shaft is rotated at extremely high speeds (e.g., 20,000-160,000 rpm). The abrasive surface of the burr scrapes against the occluding tissue and disintegrates it, reducing the occlusion and improving the blood flow through the vessel. Such a method and a device for performing the method are described in, for example, U.S. Patent No. 4,990,134 to Auth., and U.S. Patent No. US5,897,566 to Shturman (the instant inventor) et al. In the Shturman device, the abrasive element is located proximally to and spaced away from the distal end of the drive shaft.

The blood supply to the heart muscle is provided by the coronary arteries which originate from the ascending aorta. These coronary arteries and their major branches lie in shallow grooves on the surface of the myocardium. Generally, coronary arteries curve around the heart, one major branch even being called the left circumflex coronary artery. Both the myocardium and the coronary arteries are covered with the thin layer of epicardium which forms the inner wall of the pericardial sac. The outer wall of the pericardial sac is formed by a parietal pericardium. The opposing walls of the pericardial sac are very lubricious and move easily with respect to each other with every heart beat. Normally, the pericardial sac contains a small quantity of pericardial fluid which also serves to reduce friction between the opposing walls of the pericardial sac.

Rotational angioplasty (atherectomy) is frequently used to remove atherosclerotic or other blocking material from stenotic (blocked) coronary arteries. Perforation of the outer curvature of the coronary artery by the abrasive burr represents a very serious complication, since arterial blood may quickly fill the pericardial sac and cause cardiac tamponade, a life threatening situation which may require open heart surgery on some occasions. Perforation of the inner curvature of the coronary artery, which is in contact with the thick myocardium, is much less dangerous and sometimes may even go unnoticed; very often it leads to leakage of blood into the heart muscle itself, causing limited hematoma without life-threatening cardiac tamponade. Therefore, there is a long-felt need to develop a rotational atherectomy device which will allow for the selective removal of blocking material from the inner curvature of the coronary artery (or similar vessel) so as to reduce or remove the risk of perforating the outer curvature of the artery.

When performing ablative atherectomy, the drive shaft on which the rotating burr is disposed is typically somewhat rigid to allow the drive shaft to transmit torque efficiently to rotate the burr. Adequate torsional rigidity of the drive shaft is usually associated with longitudinal rigidity as well; that is, the distal end section of the drive shaft tends to stay relatively straight. One drawback to this rigidity manifests itself when the drive shaft is positioned to remove plaque or other deposits at a curved portion of a blood vessel. Since the distal end section of the drive shaft tends to stay straight, when such end section is disposed in a curved portion of a vessel, the rapidly rotating abrasive burr is pressed against the outer curvature of the vessel (e.g,. coronary artery). The burr may weaken or perforate the outer curvature of the vessel, potentially leading to a life threatening complication.

Document US 2004/158270 A1 discloses a device for ablating an occlusion in a patient's blood vessel, the device comprising a flexible drive shaft for insertion into a tubular structure and an abrasive element on the drive shaft to abrade an occlusion when the drive shaft rotates.

Document US 5 314 438 A discloses an abrasive drive shaft atherectomy device, the device including a flexible, elongated drive shaft for receipt of a guide wire therein and around which the drive shaft may be rotated.

Document US 5 681 336 A discloses a rotational ablation device, the device including a guidewire having proximal and distal ends, rotational ablations means having a hollow flexible drive shaft and abrasive burr connected thereto, a flexible sheath, and a guide catheter through which the guidewire is inserted having proximal and distal ends.

Accordingly, the invention seeks to provide a rotational device for reducing or removing deposits from the interior of a tubular structure.

The invention also seeks to provide a rotational atherectomy device or a catheter which will be usable efficiently to reduce or remove deposits from the inner curvatures of curved blood vessels and other biological and non-biological tubular structures.

The invention also seeks to provide a rotational atherectomy device which will be usable safely to reduce or remove deposits on the inner walls of curved blood vessels such as coronary arteries or bypass grafts.

It is known to provide a rotational device for removing an occlusion from inside a tubular structure, the device comprising a flexible drive shaft for insertion into a tubular structure and an abrasive element on the drive shaft to abrade an occlusion when the drive shaft rotates.

A rotational device according to the present invention is characterised by at least one solid support element on the drive shaft configured so that, when the device is being used to remove an occlusion from an inner surface of a curved section of the tubular structure, the abrasive element is biased toward the inner surface by the solid support element.

It is also known to provide a rotational device comprising a flexible drive shaft having a distal end section formed from at least one helically wound wire or at least one coil spring and an abrasive element on the distal end section.

A rotational device according to another aspect of the invention is characterised by a solid support element located at the distal end of the drive shaft and abrasive element located proximal to and spaced away from the supporting element on the distal end section spaced from the abrasive element.

In a preferred embodiment, two solid support elements are disposed on the drive shaft.

Advantageously, the device comprises a distal and a proximal solid support element respectively, the distal solid support element being distal to the abrasive element on the drive shaft and, the proximal solid support element being proximal to the abrasive element on the drive shaft.

Preferably, the distance between the distal solid support element and the abrasive element and between the proximal solid support element and the abrasive element is substantially the same.

The or each solid support element conveniently includes a shoulder that cooperates with a corresponding shoulder on the drive shaft to mount the or each solid support element to the drive shaft.

In a preferred embodiment, an adhesive layer is disposed between the or each solid support element and the drive shaft.

Preferably, the or each solid support element is rounded.

The or each solid support element may be substantially spherical in shape. However, alternatively, a longitudinal cross-section of the or each solid support element may be substantially elliptical.

The abrasive element may be substantially spherical in shape. Alternatively, a longitudinal cross-section of the abrasive element is substantially elliptical.

The or each solid support element is advantageously formed from, or coated with, a material having a low coefficient of friction.

In another embodiment, the or each solid support element may be integrally formed with the drive shaft.

In this embodiment, the or each solid support element may comprise an enlarged diameter section of the drive shaft.

The enlarged diameter section can be coated with a material having a low coefficient of friction.

The rotational device according to the invention preferably includes a guidewire for insertion into a tubular structure prior to insertion of the drive shaft, the drive shaft being configured for insertion into the tubular structure over the guidewire.

According to another aspect of the invention, there is provided an atherectomy device for the removal of an occlusion from the interior wall of a blood vessel such as a coronary artery, bypass graft and other biological or non-biological tubular structures comprising a rotational device according to the invention.

It is known to provide a method of making a rotational device for removing an occlusion from inside a tubular structure, comprising a drive shaft for insertion into a tubular structure and an abrasive element on the drive shaft to abrade an occlusion when the drive shaft rotates.

Embodiments of the invention will now be described, by way of example only, in conjunction with the following drawings, in which:
Fig. 1 is a perspective view of a rotational atherectomy device incorporating the invention;
Fig. 2 is an enlarged view of the distal end section of the drive shaft of the rotational atherectomy device incorporating the invention of Fig. 1;
Fig. 3 is a side sectional view of the distal end section of the drive shaft of the rotational atherectomy device incorporating the invention taken along line 3-3 of Fig. 2;
Figs. 4-6 are successive sectional views of the device according to the invention of Fig. 2 being advanced over a guidewire and ablating deposits in a curved blood vessel;
Figs. 7-9 are successive sectional views of the device according to the invention of Fig. 2 ablating deposits in a curved blood vessel with the guidewire being withdrawn from the distal end section of the drive shaft;
Fig. 10 is a sectional view depicting minor variations in the embodiment of Figs. 2-9.
Fig. 11 is a side sectional view of an embodiment of the invention showing structure attaching the distal solid support element to the drive shaft;
Fig. 12 is an enlarged side sectional view of the structure attaching the distal solid support element to the drive shaft of Fig. 11;
Fig. 13 is a side sectional view of another embodiment of the invention showing structure attaching the distal solid support element to the drive shaft, and
Fig. 14 is an enlarged side sectional view of the structure attaching the distal solid support element to the drive shaft of Fig. 13.

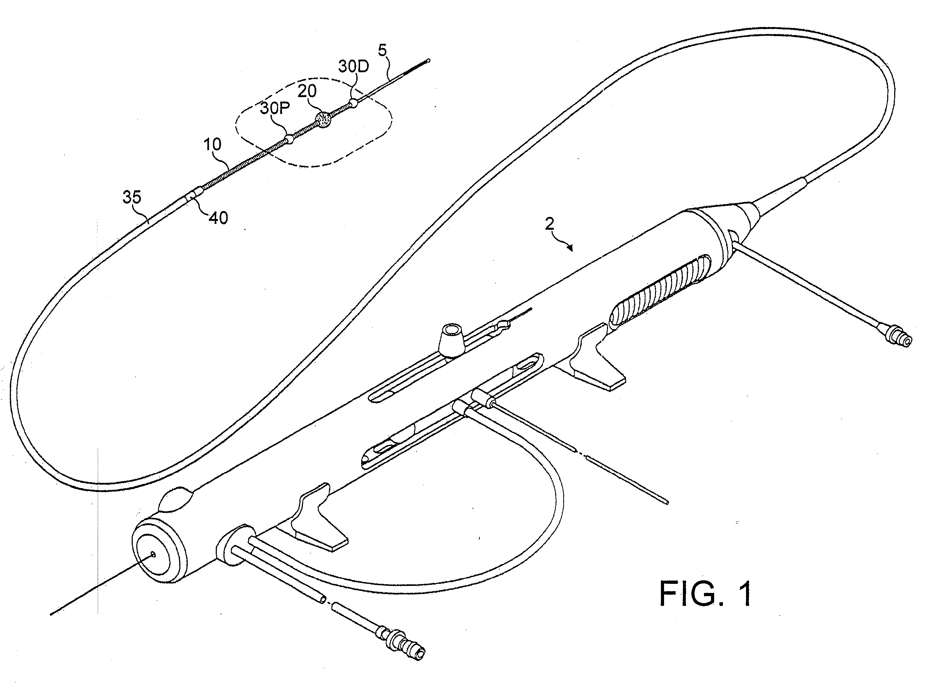

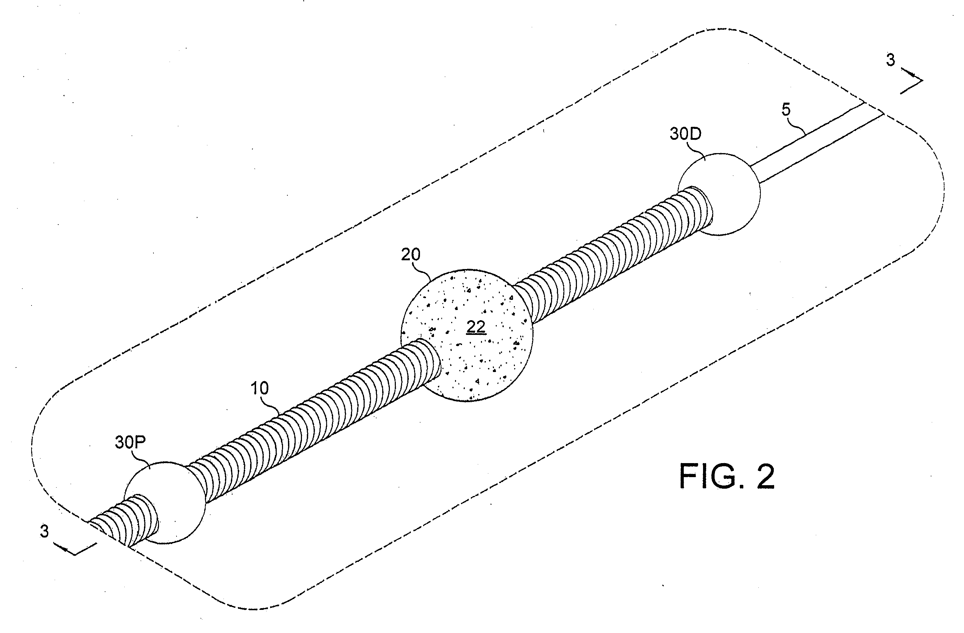

Fig. 1 is a perspective view of a rotational atherectomy device in accordance with the invention. The advancer 2 is similar to that described in the Shturman patent mentioned above. It should be understood that any type of advancer may be used, including but not limited to advancers described by Auth, other advancers described by Shturman, and advancers developed by others. The instant improvements appear in the distal end section of the drive shaft and are outlined by the dashed line box of Fig. 1 and best illustrated in Fig. 2.

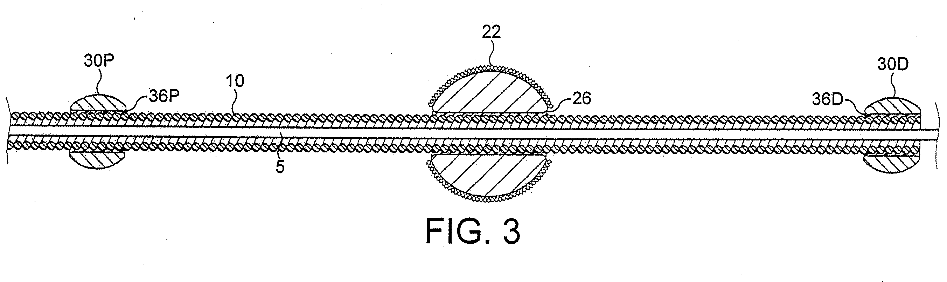

Drive shaft 10 is provided with an abrasive element (e.g. burr) 20 in its distal end section at a predetermined distance from the terminus of the drive shaft. At or near the terminus of drive shaft 10 is disposed a distal solid support element 30D. Preferably, a second proximal solid support element 30P is disposed on drive shaft 10 proximal to abrasive element 20. It is more preferable that the distance between solid support element 30P and abrasive element 20 is substantially equal to the distance between abrasive element 20 and solid support element 30D. As shown in Fig. 3, abrasive element 20 is preferably secured to drive shaft 10 via adhesive layer 26, and solid support elements 30P and D are also preferably secured to drive shaft 10 via adhesive layers 36P and 36D, respectively. However, it will be appreciated that any type of bonding such as, for example, soldering or welding may be employed to attach the support elements 30P and 30D, or the abrasive element 20 to the drive shaft 10. Abrasive element 20 has an abrasive surface 22, which may be formed by deposition of an abrasive material (e.g., diamond grit), or similar abrasive properties may be provided to the surface using laser, electrical discharge machining (EDM), or other methods of micro- or nano-machining. Solid support elements 30 are preferably made from or coated with a material having a low coefficient of friction so that they can both rapidly rotate and slide along the inner surface of a blood vessel or similar biological structure as the drive shaft is moved back and forth along the vessel without causing significant damage to the vessel wall.

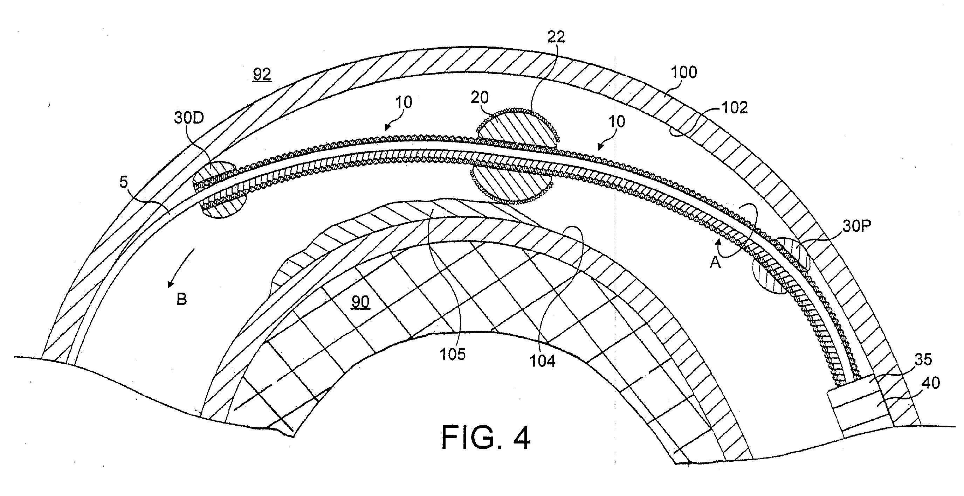

The advantages achieved by the provision of solid support elements 30 are readily understandable in view of Figs. 4-6, which depict the invention being used to reduce a partial occlusion 105 of a blood vessel 100. In all of Figs. 4-6, drive shaft 10 is being rotated within sheath 35 in the direction of arrow A and being advanced longitudinally within vessel 100 around guidewire 5 in the direction of arrow B. Without solid support elements 30, the distal end section of the drive shaft 10 would have a tendency to conform as much as possible to the outer curvature 102 of vessel wall 100 and would bring abrasive element 20 into contact with outer curvature 102. This may prove detrimental for two reasons. First, it would cause abrasive element 20 to be in poor or no contact with deposits 105 on the inner curvature 104 of vessel 100; such deposits would not be treated effectively. Moreover, the configuration which is not provided with solid support element(s) would cause abrasive element 20 to remain in contact with outer curvature 102, thereby increasing the risk of perforation of outer curvature 102 and thus the risk of blood entering the pericardial space 92. As explained above, perforation of the inner curvature 104 of an artery is less risky since the inner curvature is in contact with thick myocardial layer 90 and much less likely to lead to leaking of blood into the pericardial sac.

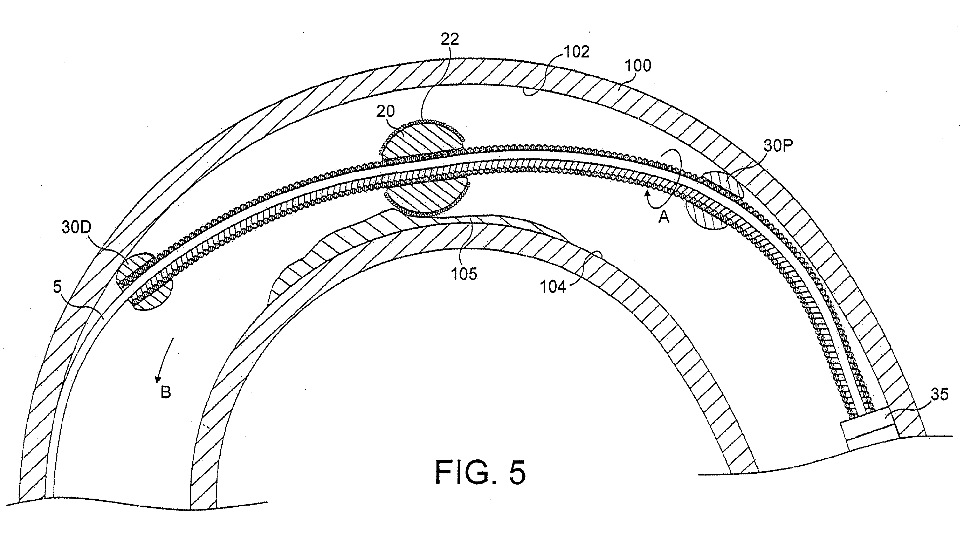

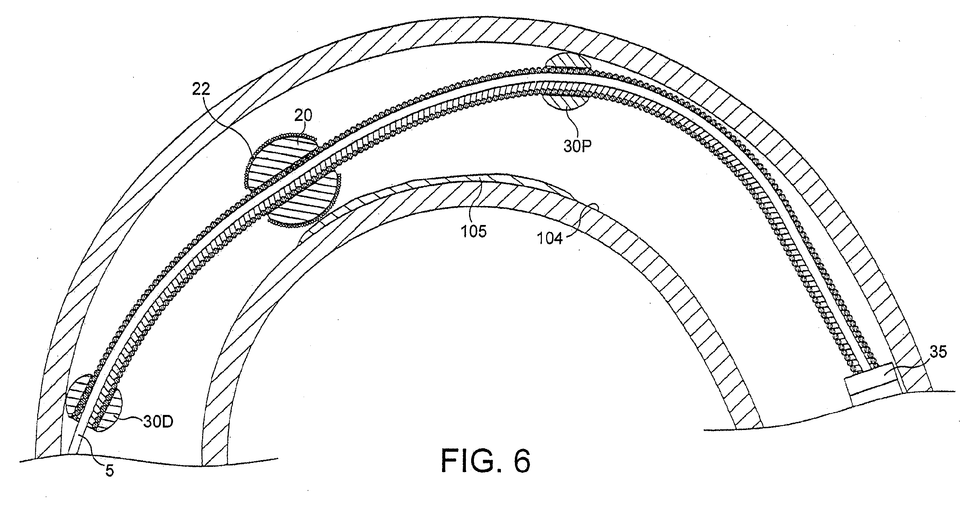

By contrast, the instant invention provides solid support elements 30 both proximal and distal of abrasive element 20. Instead of abrasive element 20 continually contacting outer curvature 102, smooth, lubricious, and atraumatic solid support elements 30 continually contact outer curvature 102. Moreover, as mentioned above, drive shaft 10 tends to remain as straight as possible. The section of drive shaft 10 between the two solid support elements 30 will be relatively straight; since the vessel curves, the central portion of this section where abrasive element 20 is disposed will be much closer to the inner curvature or surface 104 of vessel 100. Consequently, abrasive element 20 will be able to remove deposits on the otherwise harder to reach inner curvature 104 while at the same time reducing or avoiding contact with the more risky outer curvature 102, thus reducing the risk of perforation and the potential for cardiac tamponade. Fig. 4 illustrates abrasive element 20 initially contacting and abrading deposits 105, Fig. 5 illustrates rotating drive shaft 10 being advanced in the direction of arrow B and abrasive element 20 removing a portion of deposits 105, and Fig. 6 illustrates drive shaft 10 advancing further still and reducing the remaining portion of deposits 105. In order not to perforate any portion of vessel 100, the rapidly rotating abrasive element should be moved back and forth many times to gradually remove stenotic deposits. Progress should be assessed periodically during treatment by, for example, injecting contrast dye into the artery of interest. Radiopaque marker 40 positioned near the distal end of the sheath 35 allows the physician to constantly visualize the distal end of the sheath, thereby increasing the overall safety of the procedure.

Abraded particles (AP) in the embodiments of this invention travel distally along the treated vessel together with a flow of flushing fluid, blood or radiopaque solution. Abraded particles may vary in size depending on size of particles forming abrasive surface of the abrasive element, rotational speed of such abrasive element and, most importantly, the degree of uniformity of the stenotic tissue which is being removed. The less uniform is the stenotic tissue, the higher the probability that larger size particles may be produced by the rotating abrasive element and travel distally along the treated vessel. For example, irregularly calcified stenotic tissue is expected to produce abraded particles (AP) of larger size. Radiopaque solution may be injected into the treated vessel after each pass or several passes in order to appreciate progress of tissue removal and assure safety of the procedure. The position of the distal end of the sheath 35 may be better visualized by placing radiopaque marker 40 at the distal end of the sheath 35.

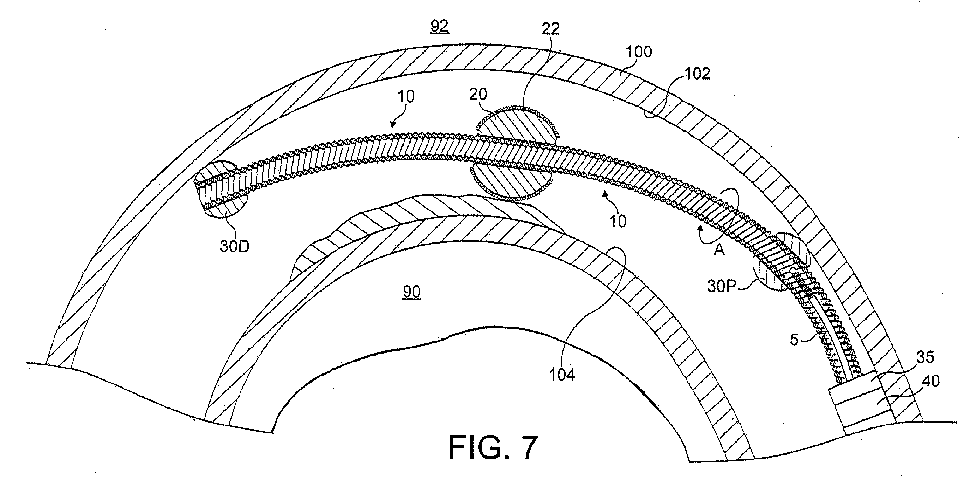

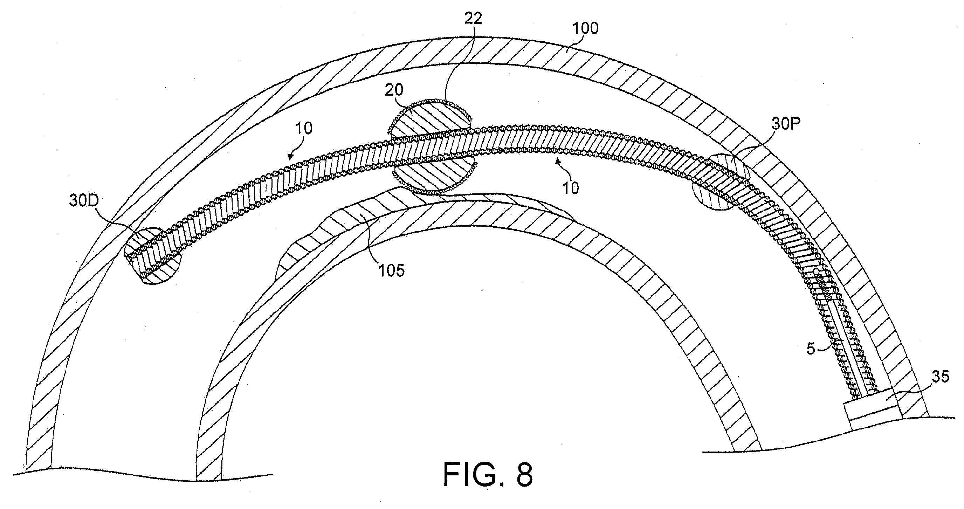

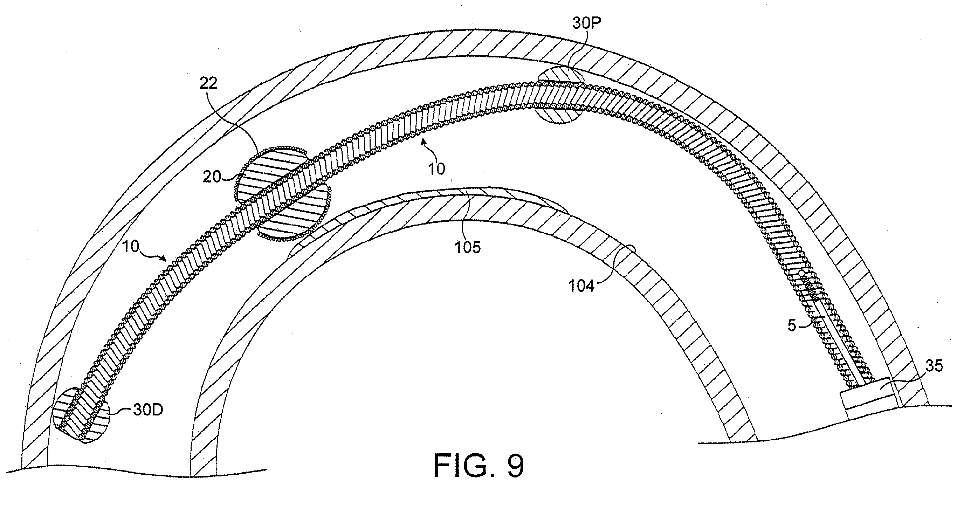

Figs. 7-9 depict essentially the same process as Figs. 4-6 but in which the guidewire 5 has been withdrawn into the lumen of the drive shaft 10 such that the distal end of the guidewire is located within the lumen of the drive shaft proximal to the distal end section of the drive shaft thereby making the distal end section of the drive shaft 10 more flexible.

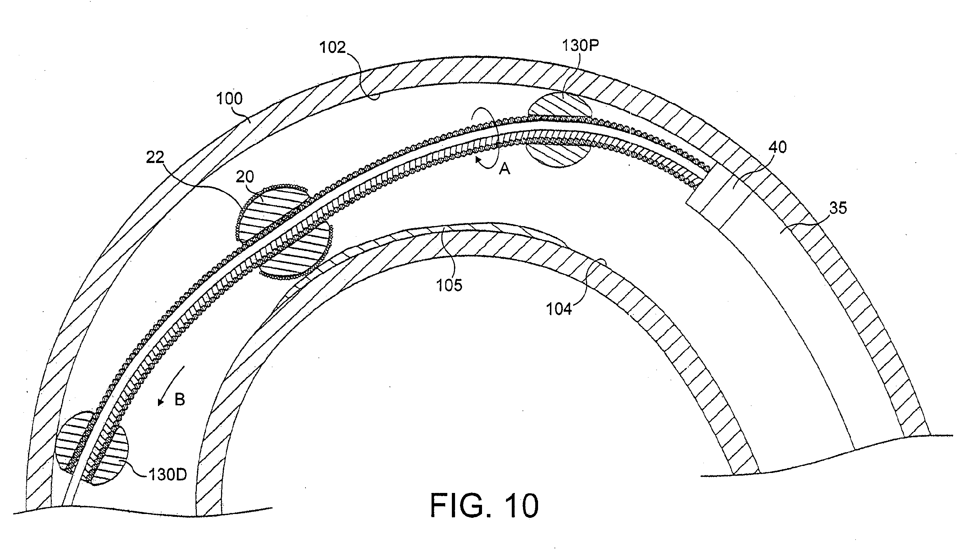

Fig. 10 depicts two slight variations in the preferred embodiment which may be employed separately or together. For example, in Figs. 4-9, drive shaft 10 is being advanced along vessel 100 from the distal end of the sheath 35, whilst sheath 35 remains in place relative to vessel 100. In the method of Fig. 10, drive shaft 10 and sheath 35 are advanced together. Also, in the embodiment of Figs. 4-9, the solid support elements 30 are significantly smaller than abrasive element 20, approximately one-half to two-thirds the diameter of abrasive element 20. However, the embodiment of Fig. 10 utilizes solid support elements 130 which are substantially the same size as, or only slightly smaller than, abrasive element 20. These larger solid support elements 130 have the effect of moving abrasive element 20 much further from outer curvature 102 of vessel 100, and thus they may be used to increase pressure of the abrasive element on the inner curvature 104 of vessel 100. Alternatively, such larger solid support elements may be used to achieve the necessary pressure of the abrasive element on the inner curvature of a vessel having a larger inner diameter or radius of curvature.

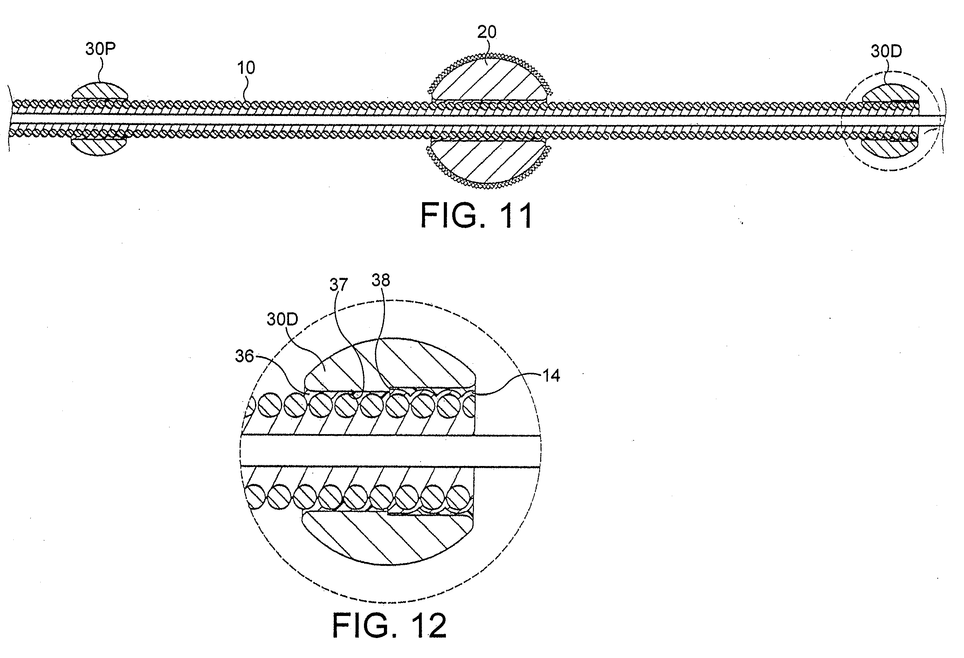

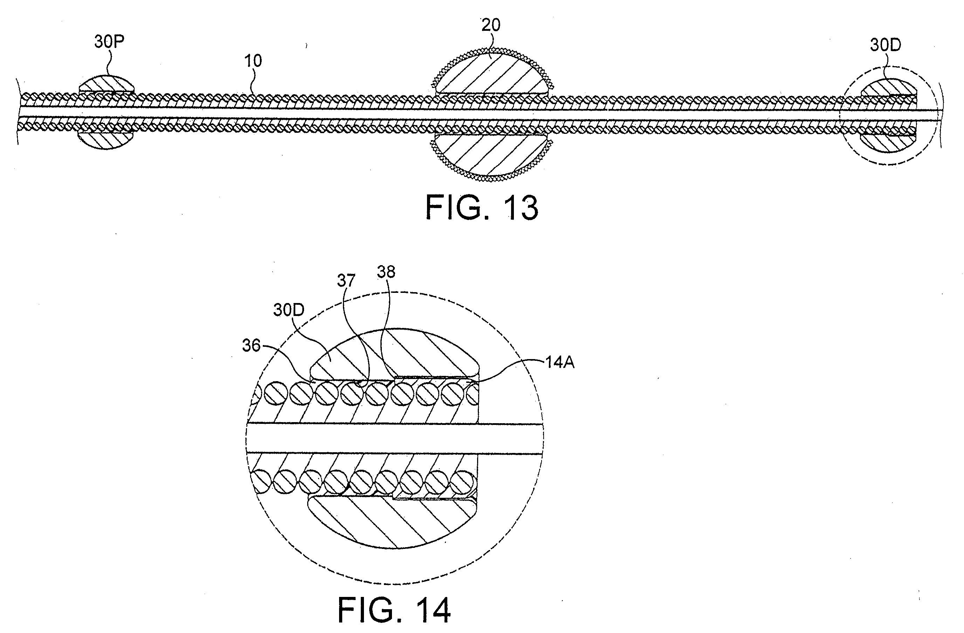

Figs. 11-14 illustrate two variations in the means by which distal solid support element 30D is secured to drive shaft 10 (Figs. 11 and 13 are the full views, while Figs. 12 and 14 are enlarged views). The solid support element is preferably provided with a shoulder or stepped portion 38 in its internal bore 37 to secure itself onto a projection or flange formed on the drive shaft 10. The variation of Figs. 11 and 12 includes a thin layer of metal 14 formed over the wrapped wires of the drive shaft 10 which conforms in profile to the coils of drive shaft wire; consequently, layer 14 is ridged. The variation of Figs. 13 and 14 is a thicker layer 14A which is flat and smooth on its outer surface. In either configuration, and in others not shown, solid support element 30D will remain securely on the distal end of drive shaft 10.

The invention is not limited to the above description. For example, the geometries of the abrasive element and solid support elements described above have been substantially spherical. However, other configurations are also contemplated. The abrasive element may be formed from windings of the drive shaft. Other shapes may be employed for the abrasive element and/or solid support element. Any convenient shape may be employed.

Further, the abrasive element and solid support elements are shown to be adhesively secured to the drive shaft. However, the solid support elements and abrasive element may be secured to the drive shaft be any known means, including soldering, welding, press-fitting, and the like. Alternatively, the solid support elements may be made of or coated with silicone or many other lubricious materials and simply formed around the drive shaft.

Also, the invention is described chiefly in context of atherectomy, however the invention is also adaptable for use to clear any arterial or venal structures. Moreover, the invention is also highly suited to clear curved arterial and arteriovenous shunts; the device will remove deposits and residue without harming the outer curvature of such shunts, which are often made of polytetrafluoroethylene (PTFE) and the like.

## Claims

1. A rotational device for removing an occlusion from inside a tubular structure, the device comprising a flexible drive shaft (10) for insertion into a tubular structure and an abrasive element (20) on the drive shaft to abrade an occlusion when the drive shaft rotates, **characterised by** a solid support element (30) on the drive shaft configured so that, when the device is being used to remove an occlusion from an inner surface of a curved section of the tubular structure, the abrasive element is biased toward the inner surface by the solid support element.

2. A rotational device comprising a flexible drive shaft (10) having a distal end section formed from at least one helically wound wire and an abrasive element (20) on the distal end section, **characterised by** a solid support element (30) on the distal end section spaced from the abrasive element.

3. A rotational device according to claim 2, comprising a distal (30D) and a proximal (30P) solid support element respectively, the distal solid support element being distal to the abrasive element on the drive shaft and, the proximal solid support element being proximal to the abrasive element on the drive shaft.

4. A rotational device according to claim 3, wherein the distal solid support element is located on the distal end of the drive shaft.

5. A rotational device according to claim 3, wherein the or each solid support element is substantially spherical in shape.

6. A rotational device according to claim 3, wherein the abrasive element is substantially spherical in shape.

## Patentansprüche

1. Rotationsvorrichtung zum Entfernen einer Okklusion vom Inneren einer rohrförmigen Struktur, wobei die Vorrichtung aufweist einen flexiblen Antriebsschaft (10) zum Einsetzen in eine rohrförmige Struktur und ein Abtragungselement (20) an dem Antriebsschaft, um eine Okklusion abzutragen, wenn der Antriebsschaft rotiert, **gekennzeichnet durch** ein Festkörper-Stützelement (30) an dem Antriebsschaft, das derart eingerichtet ist, dass, wenn die Vorrichtung verwendet wird, um eine Okklusion von einer Innenfläche eines gekrümmten Abschnitts der rohrförmigen Struktur zu entfernen, das Abtragungselement von dem Festkörper-Stützelement zu der Innenfläche hin gezwungen wird.

2. Rotationsvorrichtung mit einem flexiblem Antriebsschaft (10), der einen distalen Endabschnitt hat, der von wenigstens einem wendelförmig gewundenen Draht ausgebildet ist, und einem Abtragungselement (20) an dem distalen Endabschnitt, **gekennzeichnet durch** ein Festkörper-Stützelement (30) an dem distalen Endabschnitt, das von dem Abtragungselement im Abstand angeordnet ist.

3. Rotationsvorrichtung gemäß Anspruch 2, mit einem distalen (30D) und einem proximalen (30P) Festkörper-Stützelement, wobei das distale Festkörper-Stützelement distal zu dem Abtragungselement an dem Antriebsschaft ist und wobei das proximale Festkörper-Stützelement proximal zu dem Abtragungselement an dem Antriebsschaft ist.

4. Rotationsvorrichtung gemäß Anspruch 3, wobei das distale Festkörper-Stützelement an dem distalen Ende des Antriebsschafts angeordnet ist.

5. Rotationsvorrichtung gemäß Anspruch 3, wobei das oder jedes Festkörper-Stützelement im Wesentlichen kugelförmig in der Gestalt ist.

6. Rotationsvorrichtung gemäß Anspruch 3, wobei das Abtragungselement im Wesentlichen kugelförmig in der Gestalt ist.

## Revendications

1. Dispositif rotatif pour retirer une occlusion de l'intérieur d'une structure tubulaire, le dispositif comprenant un arbre d'entraînement souple (10) pour l'insertion dans une structure tubulaire et un élément abrasif (20) sur l'arbre d'entraînement afin d'abraser une occlusion lorsque l'arbre d'entraînement tourne, **caractérisé par** un élément de support solide (30) sur l'arbre d'entraînement configuré de sorte que, lorsque le dispositif est utilisé pour retirer une occlusion d'une surface interne d'une section incurvée de la structure tubulaire, l'élément abrasif est sollicité vers la surface interne par l'élément de support solide.

2. Dispositif rotatif comprenant un arbre d'entraînement souple (10) ayant une section d'extrémité distale formée à partir d'un fil enroulé de manière hélicoïdale et un élément abrasif (20) sur la section d'extrémité distale, **caractérisé par** un élément de support solide (30) sur la section d'extrémité distale espacée de l'élément abrasif.

3. Dispositif rotatif selon la revendication 2, comprenant un élément de support solide distal (30D) et un élément de support solide proximal (30P) respectivement, l'élément de support solide distal étant à distance de l'élément abrasif sur l'arbre d'entraînement et l'élément de support solide proximal étant à proximité de l'élément abrasif sur l'arbre d'entraînement.

4. Dispositif rotatif selon la revendication 3, dans lequel l'élément de support solide distal est positionné sur l'extrémité distale de l'arbre d'entraînement.

5. Dispositif rotatif selon la revendication 3, dans lequel le ou chaque élément de support solide a une forme sensiblement sphérique.

6. Dispositif rotatif selon la revendication 3, dans lequel l'élément abrasif a une forme sensiblement sphérique.
